# EUROPEAN PATENT APPLICATION

(11) **EP 1 484 079 A1**
(43) Date of publication of application: **08.12.2004**
(21) Application number: 03743983.3
(22) Date of filing: 20.02.2003
(51) Int. Cl.: A61M 29/02, A61F 2/06

(54) **STENT FOR INTRACRANIAL VASCULAR THERAPY AND PROCESS FOR PRODUCING THE SAME**

(30) Priority: 11.03.2002 JP 2002066185
(71) Applicant: Kaneka Corporation, Osaka-shi, Osaka 530-0005 (JP)
(72) Inventor: IWATA, Hiroo, Mishima-gun, Osaka 618-0024 (JP); NISHIDE, Takuji, Otsu-shi, Shiga 520-0105 (JP)
(74) Representative: Gille Hrabal Struck Neidlein Prop Roos
(86) International application number: PCT/JP2003/001841
(87) International publication number: WO 2003/075998

(57) **Abstract**

The present invention provides a stent for intracranial vascular therapy which can be safely held in the intracranial arteries, induces no biological reaction in the blood vessels due to galvanic corrosion or the like, and has elevated visibility under X-ray radioscopy.

A stent of the present invention includes a plurality of main struts and a plurality of link struts as its constituents, wherein the stent is made of a single material having higher radiopacity than that of stainless steel, and the main struts and the link struts each have a width ranging from 100 µm to 200 µm and a thickness ranging from 50 µm to 100 µm.

## Description

### Technical Field

The present invention relates to a stent used for intracranial vascular therapy, and more specifically to a stent used for dilation therapy of a stenosed region formed in the intracranial blood vessels, and for coil embolization therapy of an aneurysm formed in the intracranial blood vessels.

### Background Art

There is an increasing tendency to relieve a patient's pain and load in a surgical operation and to give importance to quality of life (QOL). Also, in combination with the progress of the aging of society, a conventional indiscriminate standard surgical operation has been converted to a reduced surgical operation, and percutaneous less-invasive therapy such as an endoscopic operation or the like has been regarded as important, thereby making wide use of some percutaneous therapies.

An example of therapies for angina pectoris and myocardial infarction derived from arteriosclerosis or the like is percutaneous angioplasty in which a stenosed region of the cardiac coronary arteries is dilated and recanalized by a balloon catheter inserted from outside of the body. The percutaneous therapy using a balloon catheter does not include surgical incision of an affected part and thus causes little damage to the tissue and makes an early recovery, as compared with a conventional surgical by-pass operation. Also, the percutaneous therapy requires a short time for the therapy and significantly decreases a load on a patient.

However, dilation therapy of a stenosed region of the cardiac coronary arteries using a balloon catheter has the large problem of causing restenosis in about 40% of patients after the passage of about 3 months to 6 months from the therapy. In order to decrease restenosis, the use of a stent has been studied.

A stent is a medical device which is held in a stenosed region formed in the vessels in living organisms, such as the blood vessels, the esophagus, the trachea, the urethra, the bile duct, and the like, for effectively securing the lumen. The stent in a wound state is introduced in a vessel and placed in a desired stenosed region, and then it is expanded to predetermined dimensions and held in the stenosed region.

Many clinical tests clarified that restenosis can be significantly decreased by holding a stent in a stenosed region of the coronary arteries in comparison to dilation therapy using only a balloon catheter (The New England Journal of Medicine, 1994, 331, 489-495). The usefulness of a stent has been confirmed with improvements in a method of administration before and after holding of the stent, and stents are now widely used all over the world (The New England Journal of Medicine, 1996, 334, 1084-1089). Stents are roughly divided into coil stents and slotted tube stents according to structures.

A coil stent comprises a single wire coil or a plurality of wire coils bonded together by laser welding or the like according to demand. The coil stent has the advantage that it has excellent flexibility, can easily reach a tortuous blood vessel, and causes little obstruction in the side branches. However, the coil stent has the disadvantage of slightly low radial force. A slotted tube stent comprises a tube-shaped base material having openings formed therein by laser processing or the like. Although the slotted tube stent has the advantage of high radial force, it has the disadvantage that it has relatively low flexibility and easily causes obstruction in the side branches. However, the designs of both the coil stent and the slotted tube stent can be improved to satisfy both the flexibility and radial force, and thus both stents are widely used.

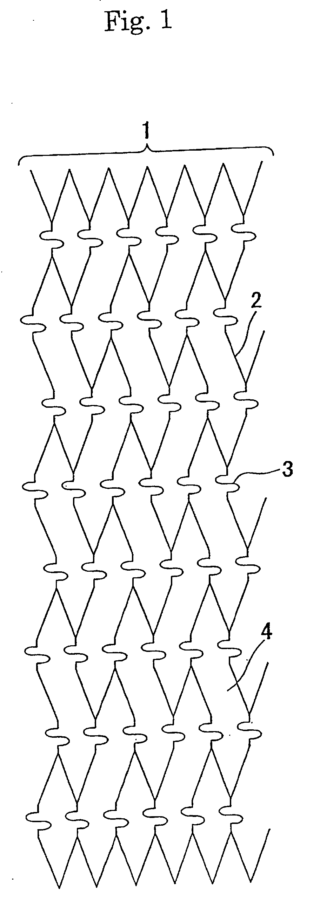

Fig. 1 is an expansion plan view of a typical stent. A stent 1 generally comprises, as its constituents, main struts 2 greatly contributing to the expression of radial force, link struts 3 greatly contributing to the expression of flexibility, and openings (cells 4) defined by the main struts 2 and the link struts 3. The radial force and flexibility of the stent 1 are determined by the shapes and dimensions of the main struts 2, the link struts, 3 and the cells 4.

Furthermore, stents are roughly divided into the following two types according to the mechanisms for expanding stents to the predetermined dimensions: One of the types is a self-expandable stent comprising a shape-memory alloy and being expandable without mechanical expansion. The other type is a balloon-expandable stent requiring mechanical expansion and being expanded by a known balloon catheter generally used for dilation therapy of a stenosed region of the vessels, particularly the arteries or the veins.

As a therapeutic method for an aneurysm formed in the intracranial blood vessels, a clipping operation has been conventionally conducted in which the root of an aneurysm is clipped in craniotomy, for preventing rupture of the aneurysm. In recent years, as less-invasive therapy, coil embolization therapy has been increasingly popularized in which an aneurysm is embolized by a metal coil percutaneously inserted from outside the body, for preventing rupture. Particularly, medical checkups of the brain are popularized, and thus aneurysms are often discovered in checkups. Most of such patients have no subjective symptom, and the coil embolization therapy having lower invasion than a conventional clipping-operation requiring craniotomy is useful for the patients.

However, it is pointed that coil embolization therapy of intracranial aneurysms has the large problem of limiting applicable cases. Namely, it is pointed out that there is the danger that a coil held in an aneurysm such as a wide neck aneurysm or a fusiform aneurysm having a large width at its root easily migrates to the parent blood vessel, and a thrombus formed in the migrating coil is dispersed by the bloodstream to cause cerebral infarction.

In order to decrease the danger of coil migration from an aneurysm, placement of a stent before or after coil embolization has been studied. Since stents for intracranial vascular therapy have not yet been applied to clinical treatments, stents for the cardiac coronary arteries are mainly used. Although the effect of preventing coil migration by using a stent is being confirmed, the following new problems are pointed out.

First, it is difficult to safely hold a stent for the cardiac coronary arteries in the intracranial arteries. This is possibly due to the anatomical characteristics of the intracranial arteries comprising a plurality of arteries. The anatomical characteristics of the intracranial arteries will be described in detail below.

The right and left vertebral arteries reaching the skull along the cervical vertebra are combined together to form the basilar artery in the skull. The basilar artery runs forward and branches into the right and left posterior cerebral arteries. On the other hand, the internal carotid arteries reaching the skull pass through the carotid artery siphon and branches into the posterior communicating artery in the occipital region, the middle cerebral artery in the temporal region, and the anterior cerebral arteries in the frontal region, and the right and left anterior cerebral arteries are connected by the anterior communicating artery. Namely, the right and left artery groups are connected in the order of the basilar artery, the posterior communicating artery, the anterior cerebral arteries, and the anterior communicating artery from the occipital region to the frontal region to form the arterial circle of cerebrum (circle of Willis). It is known that most of aneurysms causing bleeding sources of subarachnoidal hemorrhage occur in the circle of Willis. In a percutaneous approach to an aneurysm formed in the circle of Willis, it is necessary to pass through the carotid artery siphon from the internal carotid arteries or pass through the basilar artery from the vertebral arteries, and it is also necessary to track the blood vessels having complicated tortuousness in this process. A conceivable main cause of difficulty in safely holding a stent for the cardiac coronary arteries is that the stent for the cardiac coronary arteries cannot track the anatomically complicated tortuousness of the intracranial arteries.

The cardiac coronary arteries are elastic arteries rich in elasticity, and are significantly affected by the heartbeat. Also, the cardiac coronary arteries have a certain degree of tortuousness, and thus a stent for the cardiac coronary arteries is designed to balance the radial force and flexibility. On the other hand, the intracranial arteries are muscular arteries and are little affected by the heartbeat because they are far from the heart. Therefore, it is easily understood that although the radial force of a stent used for the intracranial vessels is not so important, the flexibility for allowing the stent to track the carotid artery siphon and the circle of Willis having a higher degree of tortuousness than the cardiac coronary arteries is very important.

Second, the stent for the cardiac coronary arteries held in the intracranial arteries has low visibility under X-ray radioscopy. Low visibility causes difficulty in precisely holding the stent in a target site and difficulty in obtaining information of the properties of the held stent in each diagnosis after the operation, thereby causing difficulty in obtaining a sufficient therapeutic effect. In observing the stent held in the intracranial arteries under X-ray radioscopy, the stent is observed through the skull regardless of the observation angle, and thus the visibility is possibly decreased by the influence of calcium contained in abundance in the bone components.

Therefore, it can be said that a limit in the application of a stent for the cardiac coronary arteries to the intracranial arteries is recognized in common to clinical sites. On the basis of the above-described background, prior arts relating to a stent for the intracranial blood vessels are disclosed.

PCT Japanese Translation Patent Publication No. 2001-504717 discloses a rolled sheet stent and stent catheter used for intracranial therapy. In this prior art, the rolled sheet stent is releasably mounted on the outer periphery of the distal tip of a catheter, and is held by a non-sliding release mechanism. The non-sliding release mechanism is operated remotely from the base end of the catheter so that the sheet stent can be held in a desired site. Although the sheet stent is self-expandable and comprises a plurality of cut slots for securing flexibility before or after expansion, the sheet stent essentially has an imperforate wall during expansion.

It is generally known that when a stent is held in a blood vessel, a thrombus is formed around the stent, and then the stent is covered with a neointimal membrane composed of the smooth-muscle cells and the endothelial cells as constituents which are proliferated by replacement of the thrombus. As disclosed in the above publication, when the sheet stent which becomes imperforate is held in the blood vessels, the thrombus formed around the stent may propagate downward in the blood vessels to cause the danger of acute obstruction.

Furthermore, Elgiloy composed of a cobalt-chromium-nickel alloy, Nitinol composed of a nickel-titanium alloy, stainless steel, and the like are disclosed as materials for the sheet stent. It is further disclosed that in order to increase visibility under X-ray radioscopy, the sheet stent is covered with a radiopaque material such as tin, tantalum, or the like, or a radiopaque maker comprising tantalum, platinum or gold is mounted on the sheet stent.

It is generally known that when different metals are in contact with each other under a wetting condition, corrosion referred to as bimetallic corrosion (galvanic corrosion) occurs. This corrosion is due to the fact that the metals function as a cathode and an anode, respectively, and are electrochemically eluted to locally produce a current.

Therefore, as disclosed in the prior art, when the sheet stent composed of a metal material is covered with a different metal or provided with a marker composed of a different metal for increasing visibility under X-ray radioscopy, galvanic corrosion occurs, and thus inflammatory response quite possibly occurs in the blood vessels. Although the inflammatory response is known to cause vascular obstruction, restenosis, or the like, the prior art has no consideration of this point.

### Disclosure of Invention

Accordingly, in consideration of the above-described problems, an object of the present invention is to provide a stent for intracranial vascular therapy and a process for producing the same which has flexibility for allowing the stent to be safely held in the intracranial arteries, induces no biological reaction in the blood vessels due to galvanic corrosion or the like, and has elevated visibility under X-ray radioscopy.

In order to achieve the object, a stent of the present invention which is held in the intracranial blood vessels for intracranial vascular therapy comprises a plurality of main struts and a plurality of link struts as its constituents. The stent is made of a single material having higher radiopacity than that of stainless steel, and the main struts and the link struts each have a width ranging from 100 µm to 200 µm and a thickness ranging from 50 µm to 100 µm.

The single material having higher radiopacity than that of stainless steel is preferably a metal, and more preferably gold or platinum.

A process for producing the stent which is held in the intracranial blood vessels for intracranial vascular therapy and comprises a plurality of main struts and a plurality of link struts as its constituents each having an outer surface, an inner surface, and sides comprises a step (step a) of forming a copper layer on each of the outer surfaces, the inner surfaces, and the sides of main struts and link struts constituting a stent made of stainless steel; a step (step b) of burying the stent in a thermoplastic resin material so as to expose only the outer surfaces of the main struts and link struts coated with the copper layers; a step (step c) of forming single material layers having higher radiopacity than that of stainless steel on the outer surfaces of the struts coated with the copper layers; a step (step d) of removing the thermoplastic resin material; a step (step e) of removing the copper layers; and a step (step f) of detaching the single material layers having higher radiopacity than that of stainless steel from the stent made of stainless steel to prepare the stent for intracranial vascular therapy.

The copper layers and/or the single material layers having higher radiopacity than that of stainless steel are preferably formed by plating, and more preferably electrolytic plating.

### Brief Description of the Drawings

Fig. 1 is an expansion plan view of an example of a stent for intracranial vascular therapy according to the present invention.

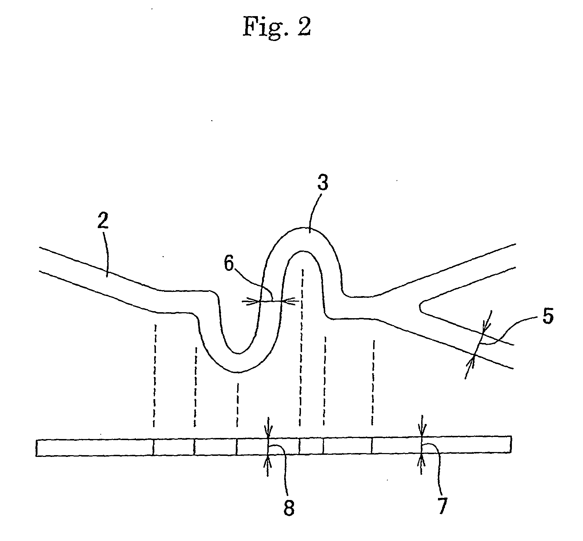

Fig. 2 is a drawing including a partial enlarged front view and side view of a stent for intracranial vascular therapy according to the present invention.

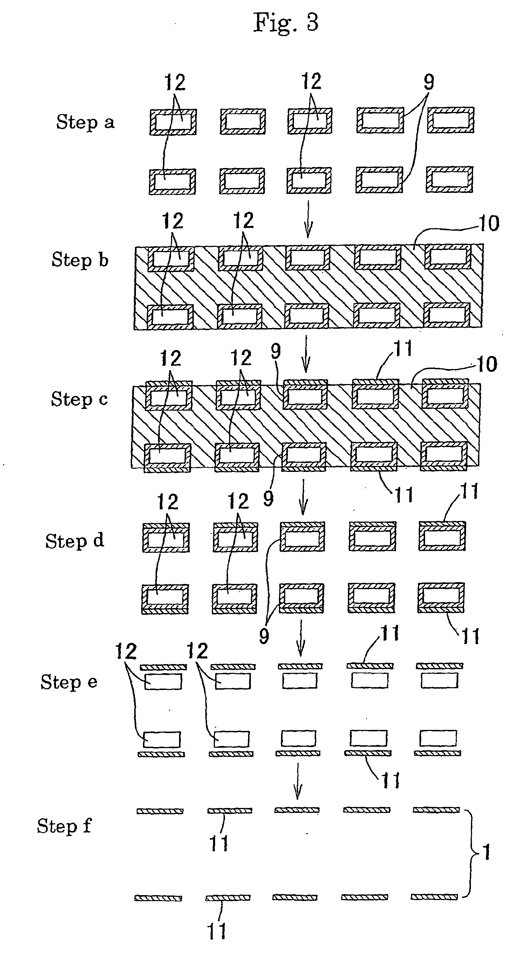

Fig. 3 is a schematic drawing showing a process for producing a stent for intracranial vascular therapy according to the present invention.
Fig. 4 is a schematic drawing of an experimental system for evaluating flexibility of a stent for intracranial vascular therapy according to the present invention.
Fig. 5 is a schematic drawing of an experimental system for evaluating visibility of a stent for intracranial vascular therapy according to the present invention.

### Best Mode for Carrying Out the Invention

A stent for intracranial vascular therapy and a process for producing the same according to embodiments of the present invention will be described in detail below.

As shown in Fig. 1, the structure and the design of a stent 1 for intracranial vascular therapy of the present invention are not limited as long as the stent 1 comprises a plurality of main struts 2, and a plurality of link struts 3 as its constituents. Namely, although the stent may be a coil stent or a slotted tube stent, the slotted tube stent is preferred because the width 5 of the main struts, and the width 6 of the link struts, the thickness 7 of the main struts, and the thickness 8 of the link struts can be easily controlled.

A mechanism for expanding the stent 1 to predetermined dimensions is also not limited. Namely, the stent 1 may be a self-expandable stent or a balloon-expandable stent.

The stent 1 for intracranial therapy of the present invention comprises the plurality of main struts 2 and the plurality of link struts 3 as its constituents, and the stent 1 is made of a single material 11 having higher radiopacity than that of stainless steel. Also, the main struts 2 and the link struts 3 each have a width ranging from 100 µm to 200 µm and a thickness ranging from 50 µm to 100 µm.

At present, stainless steel (SUS316L) is mainly used as a material for stents for the cardiac coronary arteries which can be diverted to intracranial vascular therapy. As described above, when the stent for the cardiac coronary arteries is diverted to the intracranial blood vessels, there are the two disadvantages including the disadvantage that the stent cannot be safely held in the intracranial blood vessels because of its low trackability to the tortuous intracranial blood vessels, and the disadvantage that the stent exhibits low visibility in the intracranial blood vessels under X-ray radioscopy. However, in consideration of the fact that stainless steel (SUS316L) is mainly used as a material for a stent for the cardiac coronary arteries, a material having higher radiopacity than that of stainless steel must be used for the stent for intracranial vascular therapy of the present invention.

When stainless steel is used as a material for the stent 1 for intracranial vascular therapy, of course, the visibility of the stent in the intracranial blood vessels can be improved by increasing the thicknesses of the struts in comparison to the stent for the cardiac coronary arteries. However, it can easily be imagined that an increase in the thicknesses of the struts inevitably decreases the flexibility of the stent to decrease the trackability to the tortuous intracranial blood vessels. It is thus important to use a material having higher X-rat radiopacity than stainless steel.

The term "single" is defined not only as one type of material not containing other materials but also as a material without complete or partial coating. (However, for a metal, the term "single" means not only that the material comprises a single element but also that the material may comprise a single alloy. On the other hand, in the method of adding a radiopaque material to any one of various plastic materials as described below, exceptionally, only the number of the radiopaque materials used is counted, and a type of radiopaque material may be used.) Namely, the present invention does not include a radiopaque coating or a radiopaque marker provided on the stent for improving radiopacity, the coating or the marker having a composition different from the stent composition. The reason for this will be described in detail below.

In general, the radiopaque coating or the radiopaque marker mainly comprises a metal. When the radiopaque coating or the radiopaque marker is provided on the stent for intracranial vascular therapy comprising a metal, different metals are joined together. As a result, bimetallic corrosion known as galvanic corrosion occurs under the wetting conditions such as in the blood vessels. The corrosion is due to electrochemical elution of the metals, and it induces inflammatory response in the blood vessels to quite possibly cause vascular obstruction or restenosis. Therefore, in order to prevent such response in the blood vessels, the stent 1 for the intracranial vascular therapy preferably comprises the single material 11.

The material of the stent 1 for intracranial vascular therapy is preferably a metal, more preferably a metal having a density of 13 g/cm³ or more, and most preferably gold or platinum. Although higher radiopacity than that of stainless steel can be realized by mixing any of various plastic materials and a fine powder of a barium compound, tantalum, gold, platinum, silver, or the like, the use of such a plastic material for forming the stent complicates the production process and causes decreasing the dimensional accuracy of the completed stent. Therefore, the material for the stent for intracranial vascular therapy is preferably a metal. Since the stent is permanently held in the blood vessels, gold or platinum inducing substantially no biological reaction is more preferred. Since gold or platinum has very high radiopacity, it is a preferred material from the viewpoint of increasing visibility in the skull.

For the plurality of main struts 2 and the plurality of link struts 3 which constitute the stent 1 for intracranial vascular therapy of the present invention, the main struts 2 and the link struts 3 each have a width ranging from 100 µm to 200 µm and a thickness ranging from 50 µm to 100 µm. When the width and the thickness of each of the main struts 2 and the link struts 3 are controlled in the above ranges, the stent 1 for intracranial vascular therapy can be designed for dilation therapy of a stenosed region and the purpose of preventing migration of an embolization coil, or the stent 1 for intracranial vascular therapy can be designed for a treated area such as the basilar artery, the circle of Willis, or the carotid artery siphon.

A first required characteristic for the stent for intracranial vascular therapy is flexibility allowing the stent to track the highly tortuous blood vessels. However, a certain degree of radial force is required for dilating a stenosed region and preventing migration of the embolization coil. Although the radial force is greatly affected by the design of the stent, the radial force is substantially determined by a correlation between the widths and thicknesses of the main struts 2 and the link struts 3 in the case of the same design. As a result of intensive research, the inventors found that when the width of each of the main struts 2 and the link struts 3 is less than 100 µm, or the thickness of each of the main struts 2 and the link struts 3 is less than 50 µm, the stent has the tendency that the flexibility is increased, while the radial force is relatively decreased, and thus the stent is unsuitable as the stent 1 for intracranial vascular therapy. It was also found that when the width of each of the main struts 2 and the link struts 3 is 200 µm or more, or the thickness of each of the main struts 2 and the link struts 3 is 100 µm or more, the stent has the tendency that the radial force is increased, while the flexibility is relatively decreased, and thus the stent is unsuitable as the stent 1 for intracranial vascular therapy.

The stent 1 for intracranial vascular therapy of the present invention is held in the intracranial blood vessels for intracranial vascular therapy and comprises the plurality of main struts 2 and the plurality of link struts 3 as its constituents each having an outer surface, an inner surface, and sides. The process for producing the stent 1 comprises a step (step a) of forming a copper layer 9 on each of the outer surfaces, the inner surfaces, and the sides of the main struts and the link struts constituting a stent 12 made of stainless steel, a step (step b) of burying the stent in a thermoplastic resin material 10 so as to expose only the outer surfaces of the main struts and link struts coated with the copper layers 9, a step (step c) of forming the single material layers 11 having higher radiopacity than that of stainless steel on the outer surfaces of the struts coated with the copper layers 9, a step (step d) of removing the thermoplastic resin material 10, a step (step e) of removing the copper layers 9, and a step (step f) of removing the single material layers 11 having higher radiopacity than that of stainless steel from the stent 12 made of stainless steel to prepare the stent 1 for intracranial vascular therapy. The copper layers 9 and/or the single material layers 11 having higher radiopacity than that of stainless steel are preferably formed by plating, and more preferably electrolytic plating.

In the process for producing the stent 1 for intracranial vascular therapy of the present invention, the stent 12 made of stainless steel is used as a template, and the width and thickness of each of the main struts 2 and the link struts 3 of the stent 1 for intracranial vascular therapy can be freely controlled by controlling the thicknesses of the copper layers 9 and the single material layers 11 having higher radiopacity than that of stainless steel. This control can facilitate the production of the stent 1 for intracranial vascular therapy for dilation therapy of a stenosed region and the purpose of preventing migration of the embolization coil, or the stent 1 for intracranial vascular therapy for a treated area such as the basilar artery, the circle of Willis, and the carotid artery siphon.

The step a preferably comprises plating, and more preferably electrolytic plating. Although plating types include electrolytic plating, chemical plating, hot-dip plating, spray plating, evaporation plating, vapor plating, powder plating, and the like, electrolytic plating is preferred from the viewpoint of versatility and ease of control of plating thicknesses.

The copper layers can easily be formed by electrolytic plating using the stainless steel stent 12 as a cathode and a copper plate or platinum-plated titanium as an anode in an acid copper plating bath containing 200 to 240 g/l of copper sulfate and 30 to 65 g/l of sulfuric acid. The bath temperature is preferably 20 to 50°C, and the bath is preferably stirred. Also, the current density is preferably 2 to 8 A/dm², and the thickness of the copper layers can be controlled by controlling the bath temperature, the current density, and the plating time. The composition of the plating bath is not limited to the above-described composition, and a known plating bath composition, or any one of various commercial plating baths is preferably used.

The resin material 10 used in the step b is not particularly limited. Any resin material can be used as long as it is thermoplastic and soluble in a specified solvent. Preferred examples of such a resin material include polyester, polyester elastomer, polyamide, polyamide elastomer, polyurethane, polyurethane elastomer, polyolefin, polyolefin elastomer, polycarbonate, and the like. Also, the method for burying the stent 12 in the resin material 10 is not limited, and a known method can be used. For example, the method for easily burying the stent 12 in the resin material 10 so as to expose only the outer surfaces of the struts comprises placing the stent 12 coated with the copper layers 9 on the outer surface of the resin-material 10, and placing a heat-shrinkable tube comprising a material which does not fuse with the resin material on the outer surface of the stent 12 and then shrinking the heat-shrinkable tube by heating to a temperature higher than the melting point of the resin material.

In the step b, it is important to expose only the outer surfaces of the struts. If the sides of the struts are partially exposed, the single material layers 11 having higher radiopacity than that of stainless steel are formed on the outer surfaces and the sides of the struts in the step c, and thus the single material layers 11 having higher radiopacity than that of stainless steel cannot be easily detached as the stent 1 for intracranial vascular therapy in the step f.

The step c preferably comprises plating, and more preferably electrolytic plating. Although plating types include electrolytic plating, chemical plating, hot-dip plating, spray plating, evaporation plating, vapor plating, powder plating, and the like, electrolytic plating is preferred from the viewpoint of versatility and ease of control of plating thicknesses.

When gold is used for the single material layers 11 having higher radiopacity than that of stainless steel, gold layers can easily be formed by electrolytic plating using the stainless steel stent 12 buried in the resin material 10 in the step b as a cathode and platinum-plated titanium as an anode in an acid bath containing 4 to 8 g/l of potassium cyanoaurate, 30 to 40 g/l of potassium citrate, 30 to 40 g/l of potassium primary phosphate, 20 to 30 g/l of citric acid, and 2 to 3 g/l of nickel ethylenediaminediacetate. The bath temperature is preferably 20 to 50°C, and the bath is preferably stirred. Also, the current density is preferably 1 to 5 A/dm², and the thickness of the gold layers can be controlled by controlling the bath temperature, the current density, and the plating time. The composition of the plating bath is not limited to the above-described composition, and a known plating bath composition, or any one of various commercial plating baths (for example, TEMPEREX 8400 produced by Electroplating Engineers of Japan Ltd.) is preferably used.

When platinum is used for the single material layers 11 having higher radiopacity than that of stainless steel, the platinum layers can easily be formed by electrolytic plating using the stainless steel stent 12 buried in the resin material 10 in the step b as a cathode and platinum-plated titanium as an anode in a phosphate bath containing 4 g/l of platinum tetrachloride pentahydrate, 20 g/l of ammonium hydrogenphosphate, and 100 g/l of sodium hydrogenphosphate dodecahydrate. The bath temperature is preferably 90 to 100°C, and the bath is preferably stirred. Also, the current density is preferably 0.3 to 1 A/dm², and the thickness of the platinum layers can be controlled by controlling the bath temperature, the current density, and the plating time. The composition of the plating bath is not limited to the above-described composition, and a known plating bath composition, or any one of various commercial plating baths (for example, Platanex 3LS produced by Electroplating Engineers of Japan Ltd.) is preferably used.

In the step d, only the resin material 10 is preferably dissolved in a good solvent for the thermoplastic resin material 10 used in the step b. When gold or platinum is used for the single material layers 11 having higher radiopacity than that of stainless steel, the metal is highly stable to various solvents, and thus a good solvent for the resin material 10 is preferably used. For example, when a polyamide elastomer comprising polyether as a soft segment and nylon 12 as a hard segment is used as the resin material 10, 1,1,1,3,3,3-hexafluoro-2-propanol can be used for removing only the resin material 10 by dissolving it within a short time.

In the step e, the copper layers 9 are preferably removed by dissolving in nitric acid. When gold or platinum is used for the single material layers 11 having higher radiopacity than that of stainless steel, any of the metals is very stable to nitric acid, and thus nitric acid is preferably used.

After each of the steps, a necessary and sufficient washing step and drying step are preferably performed. Also, electrolytic plating is performed in the steps a and c, hydrogen gas is produced by electrolysis. In the step e, hydrogen gas is produced by dissolution of the copper layers 9 in nitric acid. Any of the steps possibly causes hydrogen brittle fracture which decreases the strength of the layers due to occlusion of the produced hydrogen gas in each layer. In order to prevent such fracture, a baking treatment is preferably performed after each of the steps a, c, and e. Although, in the baking treatment after the step c, the melting point of the resin material 10 must be taken into consideration, each baking treatment is preferably performed in the range from 150 to 300°C.

Besides the above-described process for producing the stent 1 for intracranial vascular therapy of the present invention, of course, it is possible to preferably use known methods such as a method in which a pipe made of the single material 11 having higher radiopacity than that of stainless steel is processed into a stent shape by laser cutting, a method in which a wire made of the single material 11 having higher radiopacity than that of stainless steel is knitted into a stent shape, and the like.

Although examples and comparative examples of the present invention will be described in detail below, the present invention is obviously not limited to these examples.

### (Example 1)

(Step a) A slotted tube stent (diameter after expansion: 3.0 mm, length: 18 mm, the number of links in the stent length direction: 6, and the number of links in the stent circumferential direction: 6) was produced by laser-cutting stainless steel SUS316L and used as a cathode, and platinum-plated titanium was used as an anode for electrolytic plating in an acid copper plating bath at 25°C (200 g/l of copper sulfate and 50 g/l of sulfuric acid) under stirring with a magnetic stirrer with a current density of 7.8 A/dm² for 30 minutes to form copper layers to a thickness of about 10 µm on the outer surface, the inner surface and the sides of the slotted tube stent. After electrolytic plating, the stent was washed in distilled water and then dried.
(Step b) The slotted tube stent having the copper layers having a thickness of about 10 µm was placed on the outer surface of a single lumen tube (inner diameter 0.53 mm, outer diameter 1.55 mm) which was produced by extrusion molding of polyamide elastomer (trade name: PEBAX7233; elf atochem Corporation) used as a resin material, and a heat-shrinkable tube was placed on the outer surface-of the stent and then heated by hot air at a flow rate of 20 l/min at a temperature of 210°C for 30 seconds to bury the stent in the resin material.
(Step c) The slotted tube stent buried in the resin material was used as a cathode, and platinum-plated titanium was used as an anode for electrolytic plating in a gold plating bath of 65°C (TEMPEREX 8400 produced by Electroplating Engineers of Japan Ltd.) under stirring with a magnetic stirrer with a current density of 0.8 A/dm² for 60 minutes to form a gold layer to a thickness of about 55 µm on the outer surface of the slotted tube stent. After electrolytic plating, the stent was washed in distilled water and the dried. After the drying, a heat treatment was performed in an oven at 150°C for 1 hour to remove the hydrogen gas produced in electrolytic plating and occluded in the gold layer.
(Step d) The stent plated with gold was immersed in 5 ml of 1,1,1,3,3,3-hexafluoro-2-propanol (produced by Nacalai Tesque Inc., and referred to as "HFP" hereinafter), and then allowed to stand at room temperature for 12 hours to remove the polyamide elastomer by dissolving. Twelve hours after, HFP was changed by 5 ml of new HFP, and the stent was allowed to stand at room temperature for 1 hour for washing. After the washing, the stent was dried at room temperature.
(Steps e and f) Nitric acid (produced by Wako Pure Chemical Industries, Ltd.) was adjusted to a concentration of 2N, and five gold-plated stents from each of which the polyamide elastomer was removed were immersed in 5 ml of 2N nitric acid and then allowed to stand at room temperature for 12 hours to remove the copper layers by dissolving. Twelve hours after, the stents were washed with distilled water and then dried. After the drying, a heat treatment was performed in an oven at 150°C for 1 hour to remove the hydrogen gas produced by dissolution of the copper layers and occluded in the gold layers. After removal, the gold layers were detached from the slotted tube stents made of stainless steel SUS316L to prepare five stents made of only gold.

### (Example 2)

Five stents were produced by the same method as in Example 1 except that in the step c, electrolytic plating of gold was performed for 90 minutes to form gold layers of about 100 µm in thickness.

### (Example 3)

Five stents were produced by the same method as in Example 1 except that in the step c, electrolytic plating was performed in a platinum plating bath at 80°C (Platanex 3LS produced by Electroplating Engineers of Japan Ltd.) under stirring with a magnetic stirrer with a current density of 2.0 A/dm² for 300 minutes to form platinum layers of about 60 µm in thickness on the outer surfaces of the stents.

### (Comparative Example 1)

In Comparative Example 1, five slotted tube stents were produced by laser-cutting stainless steel SUS316L used in Example 1.

### (Comparative Example 2)

Five stents were produced by the same method as in Example 1 except that in the step c, electrolytic plating of gold was performed for 30 minutes to form gold layers of about 30 µm in thickness.

### (Comparative Example 3)

Five stents were produced by the same method as in Example 1 except that in the step c, electrolytic plating of gold was performed for 120 minutes to form gold layers of about 110 µm in thickness.

### (Measurement of dimensions of each part of stent)

The widths and thicknesses of three main struts and three link struts of each of the stents of the examples and comparative examples were measured by using a micro highscope (VH-7000 produced by Keyence Corporation) under the condition of a magnification of x150. The average of three measurements was regarded as a measured value, and the average and standard deviation of the three stents are shown in Table 1.

### (Evaluation of radial force)

The stent of each of the examples and the comparative examples was placed on a balloon of a balloon catheter for PTCA (percutaneous transluminal coronary angioplasty) comprising a balloon of 3.0×20 mm, and clipped. Also, a silicon tube having an inner diameter of 3 mm and an outer diameter of 6 mm was set in hot water at 37°C, and the balloon was expanded in the silicon tube to hold the stent therein (pressing rate: 0.101 MPa/sec, pressure: 0.811 MPa, and pressing time: 30 sec). After holding, the silicon tube was incised to remove each stent, and damage to the main struts and the link struts was measured for evaluating the radial force. When significant damage such as deformation or fracture due to expansion of each stent was observed in the main struts or the link struts, the radial force was decided to be insufficient. The evaluation results of three stents with n = 3 are shown in Table 1.

### (Evaluation of flexibility)

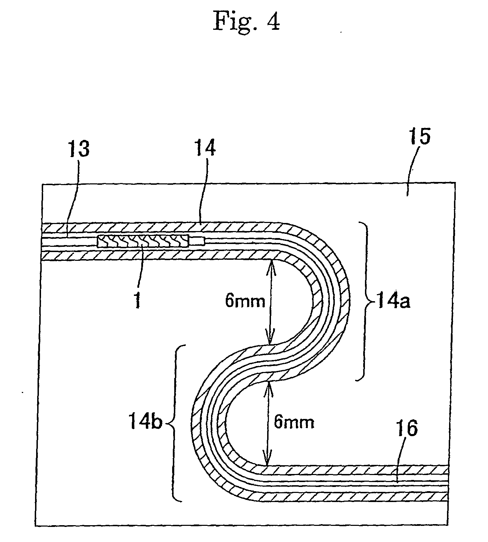

The stent of each of the examples and the comparative examples was placed on a balloon of a balloon catheter 13 for PTCA (percutaneous transluminal coronary angioplasty) comprising a balloon of 3.0×20 mm, and clipped. As shown in Fig. 4, a simulated intracranial blood vessel 14 (made of high-density polyethylene and having an inner diameter of 3 mm, an outer diameter of 6 mm, and a radius of curvature of the first and second bends of 3 mm) comprising a first bend 14a and a second bend 14b was arranged in a flexible plate 15 in hot water at 37°C, and a guide wire 16 of 0.014" (0.3556 mm) was previously inserted into the simulated intracranial blood vessel. The balloon catheter 13 clipped with each stent was inserted into the simulated intracranial blood vessel at a rate of 10 mm/sec by using a slide table, and flexibility of each stent was evaluated by determining whether the stent could be inserted or not. The evaluation results of the stents with n = 3 are shown in Table 1.

### (Evaluation of visibility)

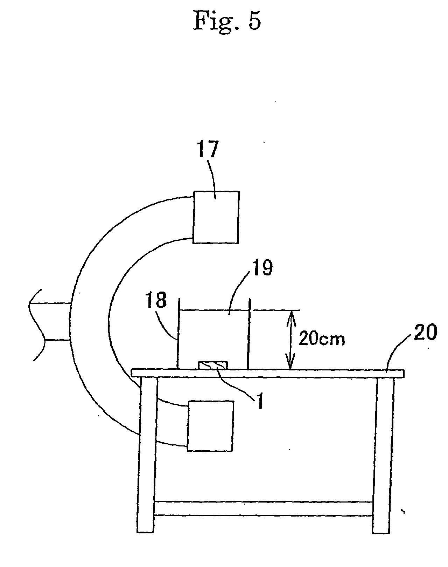

As a simulated intracranial environment, a plastic vessel 18 was filled with distilled water 19 so that the distance between the bottom of the plastic vessel and the water surface was 20 cm. As shown in Fig. 5, the stent 1 was placed at the bottom of the plastic vessel 18 set on an operating table 20, and contrast radiography was performed by using an X-ray contrast radiographic apparatus 17 (BV-300 produced by Philip Co,. Ltd.) to evaluate the visibility of each stent. The evaluation results of the stents with n = 3 are shown in Table 1.

### (Evaluation results)

**Table 1**

| Evaluation Results of Stents of Examples and Comparative Examples | | | | | | | |
|---|---|---|---|---|---|---|---|
| | Material | Thickness of main strut (µm) | Width of main strut (µm) | Width of link strut (µm) | Radial force (*1) | Flexibility (*2) | Visibility (*3) |
| Example 1 | Gold | 57±4 | 163±9 | 115±5 | ○ | ○ | ○ |
| Example 2 | Gold | 93±6 | 185±9 | 132±8 | ○ | ○ | ○ |
| Example 3 | Platinum | 65±3 | 170±6 | 121±5 | ○ | ○ | ○ |
| Comp. Example 1 | SUS316L | 117±3 | 118±5 | 69±2 | ○ | Δ | × |
| Comp. Example 2 | Gold | 31±2 | 140±7 | 94±3 | × | ○ | ○ |
| Comp. Example 3 | Gold | 110±8 | 210±8 | 144±7 | ○ | × | ○ |

| | | | | | | | |
|---|---|---|---|---|---|---|---|
| *1: ○ (No breakage occurred in the main struts and the link struts in expansion of the stent.) × (Breakage occurred in the main struts or the link struts in expansion of the stent.) | | | | | | | |
| *2: ○ (The stent could pass through both the first and second bends.) Δ (The stent could pass through the first bend and could not pass through the second bend.) × (The stent could not pass through the first bend.) | | | | | | | |
| *3: ○ (Higher visibility than a stainless steel stent) × (The same degree of visibility as a stainless steel stent) | | | | | | | |

In Examples 1 to 3 of the present invention, no damage was observed in the main struts and the link struts in expansion of the balloon, and thus a certain degree of radial force was exhibited. Also, each of the stents could pass through the first and second bends of the simulated intracranial blood vessel, and thus sufficient flexibility was exhibited. Furthermore, since gold (Examples 1 and 2) and platinum (Example 3) were used as materials, high visibility was exhibited.

In Comparative Example 1, the stent had high radial force, but it could not pass through the second bend, thereby exhibiting slightly poor flexibility. Furthermore, since SUS316L used as the material was the same as existing stents for the cardiac coronary arteries, the stent exhibited relatively low visibility.

In Comparative Example 2, it was found that the stent had high flexibility and visibility because the main struts were relatively thin and gold was used as the material. However, since the main struts were thin, the struts were cut at 3 to 5 positions per stent when the stent was expanded in the simulated blood vessel, and thus the stent exhibited insufficient radial force.

On the other hand, in Comparative Example 3, no damage was observed in the main struts and the link struts in expansion of the balloon, and thus a certain degree of radial force was exhibited. Also, the visibility was high. However, since the main struts and the link struts were wide and thick, the stent had low flexibility and could not pass through the first bend in evaluation of flexibility.

### Industrial Applicability

As described above, a stent for intracranial vascular therapy of the present invention comprises a plurality of main struts and a plurality of link struts as its constituents, the stent is made of a single material having higher radiopacity than that of stainless steel, and the main struts and the link struts each have a width ranging from 100 µm to 200 µm and a thickness ranging from 50 µm to 100 µm. Therefore, the stent for intracranial vascular therapy can be safely held in the intracranial arteries, induces no biological reaction in the blood vessels due to galvanic corrosion or the like, and has elevated visibility under X-ray radioscopy.

## Claims

1. A stent for intracranial vascular therapy comprising a plurality of main struts and a plurality of link struts as its constituents, wherein the stent is made of a single material having higher radiopacity than that of stainless steel, and the main struts and the link struts each have a width ranging from 100 µm to 200 µm and a thickness ranging from 50 µm to 100 µm.

2. The stent for intracranial vascular therapy according to claim 1, wherein the single material having higher radiopacity than that of stainless steel is a metal.

3. The stent for intracranial vascular therapy according to claim 2, wherein the metal is gold.

4. The stent for intracranial vascular therapy according to claim 2, wherein the metal is platinum.

5. A process for producing a stent for intracranial vascular therapy according to any one of claims 1 to 4 comprising a plurality of main struts and a plurality of link struts as its constituents, each of the struts having an outer surface, an inner surface, and sides, the process comprising a step (step a) of forming a copper layer on each of the outer surfaces, the inner surfaces, and the sides of the main struts and the link struts constituting a stent made of stainless steel; a step (step b) of burying the stent in a thermoplastic resin material so as to expose only the outer surfaces of the main struts and link struts coated with the copper layers; a step (step c) of forming single material layers having higher radiopacity than that of stainless steel on the outer surfaces of the struts coated with the copper layers; a step (step d) of removing the thermoplastic resin material; a step (step e) of removing the copper layers; and a step (step f) of detaching the single material layer having higher radiopacity than that of stainless steel from the stent made of stainless steel to prepare the stent for intracranial vascular therapy.

6. The process for producing the stent for intracranial vascular therapy according to claim 5, wherein the copper layers and/or the single material layers having higher radiopacity than that of stainless steel are formed by plating.

7. The process for producing the stent for intracranial vascular therapy according to claim 6, wherein the plating is electrolytic plating.
